(19) Europäisches Patentamt
European Patent Office
Office européen des brevets

(11) **EP 3 011 923 B1**

(12) **EUROPÄISCHE PATENTSCHRIFT**

(45) Veröffentlichungstag und Bekanntmachung des Hinweises auf die Patenterteilung:
**01.12.2021 Patentblatt 2021/48**

(51) Int Cl.:
*A61B 18/12* *(2006.01)*        *A61B 18/04* *(2006.01)*
*A61B 18/00* *(2006.01)*

(21) Anmeldenummer: **14190155.3**

(22) Anmeldetag: **23.10.2014**

(54) **Einrichtung zur Metallerkennung bei der Einwirkung auf biologisches Gewebe mittels eines funkenbildenden elektrochirurgischen Instruments**

Device for metal detection when acting on biological tissue by means of a spark-forming electrosurgical instrument

Dispositif de reconnaissance de métal lors d'une action sur des tissus biologiques au moyen d'un instrument électrochirurgical formant des étincelles

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**27.04.2016 Patentblatt 2016/17**

(73) Patentinhaber: **Erbe Elektromedizin GmbH**
**72072 Tübingen (DE)**

(72) Erfinder: **Keller, Dr. Sandra**
**72379 Hechingen (DE)**

(74) Vertreter: **Rüger Abel Patentanwälte PartGmbB**
**Patentanwälte**
**Webergasse 3**
**73728 Esslingen a. N. (DE)**

(56) Entgegenhaltungen:
EP-A1- 1 849 425        WO-A1-97/45157
DE-A1-102004 010 769    US-A1- 2012 215 213

**Beschreibung**

Die Erfindung betrifft eine Einrichtung zum Erkennen von Fremdkörpern, insbesondere metallischen Fremdkörpern im biologischen Gewebe bei Einwirkung auf dieses mittels elektrochirurgischer Instrumente, die elektrische Entladungen erzeugen.

Elektrochirurgische Instrumente, die eine im fluidförmigen Medium stattfindende elektrische Entladung nutzen, um auf biologisches Gewebe einzuwirken, sind prinzipiell bekannt. Solche Instrumente sind beispielsweise Argon-Plasma-Koagulationsinstrumente, die einen in Argonatmosphäre brennenden Funken oder einen Plasmastrahl erzeugen, Funkenskalpelle oder dergleichen. Monopolare und bipolare Resektionsinstrumente, die in nichtleitfähiger (Purisole) oder leitfähiger (Kochsalzlösung) Flüssigkeit, die Flüssigkeit derart erhitzen, dass die Flüssigkeit verdampft und sich in diesem Dampf Funken zum Gewebe oder einer weiteren Elektrode bilden sind bekannt. Beim Umgang mit Argon-Plasma-Koagulationsinstrumenten ist besondere Vorsicht geboten, wenn im biologischen Gewebe Fremdkörper, insbesondere metallische Fremdkörper, vorhanden sind. Es kann sich dabei beispielsweise um Stents oder sonstige Metallteile handeln, die einem Patienten bereits in der Vergangenheit oder auch während des aktuellen Eingriffs eingepflanzt worden sind. Erfolgt die Funken- oder Plasmaeinwirkung auf einen Stent, eine metallische Klammer oder einem sonstigen Metallkörper im biologischen Gewebe, unbeabsichtigt, kann daraus eine Schädigung des Metallteils folgen, wodurch dieses seine Funktion verlieren kann. Auch kann das umliegende Gewebe beispielsweise aufgrund von Wärmeleitung unerwünscht geschädigt werden.

Andererseits gibt es Fälle, in denen die Funken-oder Plasmaeinwirkung auf den metallischen Fremdkörper gewünscht ist, beispielsweise um die Kürzung eines Stents oder bestimmte chirurgische Eingriffe vorzunehmen. Solche sind beispielsweise die Koagulation eines blutenden Gefäßes mittels einer anatomischen Pinzette, die durch den Funken-oder Plasmastrahl des elektrochirurgischen Instruments gezielt erwärmt und somit zur Koagulation des Gefäßes befähigt wird.

Weiterhin ist für die Anwendung von monopolaren und bipolaren Resektionsschlingen in der TUR bekannt, dass es bei zu geringem Abstand zwischen der Resektionsschlinge und dem Resektoskop unbeabsichtigt zur Funkenbildung auf das metallische Resektoskop kommt. Diese unerwünschte Funkenbildung führt zum Stromfluss durch das metallische Resektoskop. Da das Resektoskop wiederum mit dem biologischen Gewebe in Kontakt ist, kommt es zu unerwünschten Koagulationseffekten an dem Gewebe.

Aus der US 2012/0215213 A1 sind ein Verfahren und eine Einrichtung zur Steuerung der Energiebeaufschlagung von biologischem Gewebe bei der Elektrochirurgie bekannt. Zum Trennen des Gewebes wird mit einem HF-Funken auf dieses eingewirkt. Zur Unterscheidung, ob der Funken Gewebe oder ein Metallteil trifft, wird eine den Strom kennzeichnende Spannung durch einen Hochpass geleitet und danach sowohl mit einer oberen wie auch mit einer unteren Schwelle verglichen. Die entstehenden Signale werden weiter ODERverknüpft und gefiltert.

Daraus ergibt sich die Aufgabe, ein Konzept zu schaffen, mit dem sich die Einwirkung eines funkenbildenden elektrischen Instruments auf Metall sicher von der Einwirkung des Instruments auf biologisches Gewebe unterscheiden lässt.

Diese Aufgabe wird mit der Einrichtung nach Anspruch 1 gelöst .

Die Erfindung betrifft eine Einrichtung gemäß Anspruch 1. Vorteilhafte Ausführungsformen der Erfindung ergeben sich aus den anhängigen Ansprüchen.

Aspekte, Ausführungen und Beispiele die nachstehend beschrieben werden und nicht unter den Wortlaut der Ansprüche fallen, sind nicht Teil der Erfindung.

Die erfindungsgemäße Einrichtung kann zu einem elektrochirurgischen Instrument gehören oder Bestandteil desselben sein. Sie kann ebenso gut zu einem zur Speisung des Instruments vorgesehenen Generator gehören oder Bestandteil desselben sein. Alternativ kann die Einrichtung als gesonderte Baugruppe zwischen dem Generator und dem elektrochirurgischen Instrument angeordnet sein.

Zu der Einrichtung gehört eine Messeinrichtung zur Erfassung einer von dem Generator zum Betrieb des Instruments bereitgestellten Spannung und zur Erfassung des von dem Generator zu dem Instrument gelieferten Stroms. Ist der Innenwiderstand des Generators gering oder Null und die abgegebene Spannung bekannt, kann auf die Messung derselben auch verzichtet werden; es genügt dann die Erfassung des Stroms. Die Erfassung des Stroms (und der Spannung) kann kontinuierlich oder intermittierend, beispielsweise in kurzen Zeitabständen erfolgen. Vorzugsweise erfolgt die Erfassung der Spannung und des Stroms in kurzen Zeitabständen. Liefert der Generator eine Wechselspannung zur Speisung des elektrochirurgischen Instruments, vorzugsweise eine HF-Wechselspannung, sind die Zeitabstände vorzugsweise kürzer als die halbe Periodendauer der Spannung bzw. des Stroms. Bei der Erfassung der Spannung bzw. des Stroms wird mindestens ein geeigneter Kennwert der Spannung sowie des Stroms erfasst. Ein solcher Kennwert kann der Augenblickswert, der Spitzenwert, der Mittelwert, der Effektivwert oder ein anderer zur Charakterisierung geeigneter Wert sein.

Der zur Einrichtung gehörige Metalldetektor ist dazu eingerichtet, anhand des Stroms und anhand der Spannung, d.h. letztlich anhand eines oder mehrerer Kennwerte für den Strom sowie eines oder mehrere Kennwerte für die Spannung, zu entscheiden, ob ein von dem Instrument ausgehender Funken auf biologisches Gewebe oder ein Metallteil trifft.

Mit dieser Einrichtung kann der Anwender auch bei schlechten Sichtverhältnissen zuverlässig und frühzeitig erkennen, wenn mit einem Funken oder Plasma auf ein Metallteil eingewirkt wird. Es ist möglich, den Metalldetektor mit einer Signalisierungseinrichtung zu verbinden, um dem Anwender ein geeignetes, zum Beispiel haptisches, optisches oder akustisches Signal bereitzustellen, damit dieser eine Metallberührung durch das Plasma oder den Funken erkennt. Es ist außerdem möglich, ein von dem Metalldetektor erzeugtes Signal zum Abschalten des Generators oder zu seiner anderweitigen Steuerung zu nutzen. Zum Beispiel kann die Leistung des Generators bei Metallerkennung reduziert werden, um unerwünschte biologische Wirkungen zu vermeiden. Ist hingegen eine Einwirkung auf das Metall gewünscht, kann das Signal dazu genutzt werden, den Generator nicht abzuschalten sondern in seiner Leistung zu erhöhen, wenn eine Berührung von Metall durch den Funken oder das Plasma erkannt wird, um zum Beispiel das Trennen von Metall mittels Funkens oder Plasmas zu unterstützen.

Der Metalldetektor enthält eine Analyseeinrichtunc, die eine Widerstandskenngröße und eine Funkenkenngröße ermittelt. Die Widerstandskenngröße ist ein vom Gewebewiderstand abhängiger Wert. Die Funkenkenngröße ist ein von der Funkengröße abhängiger Wert. Beide Kenngrößen, die Widerstandsgröße und die Funkenkenngröße, werden mit entsprechenden Schwellwerten verglichen, um aus diesem Vergleich ein aussagekräftiges Signal zu generieren. Erfindungsgemäß wird eine Metallberührung durch das Plasma oder den Funken dann signalisiert, wenn die elektrische Widerstandskenngröße, der Widerstandskennwert den Widerstandsschwellwert unterschreitet und die Funkenkenngröße, der Funkenkennwert den Funkengrößenschwellwert überschreitet. Alle anderen Kombinationen können anderen Situationen zugeordnet werden:

**[0015]** - Der Widerstandskennwert unterschreitet den Widerstandsschwellwert und die Funkenkenngröße ist geringer als der Funkengrößenschwellwert. Es liegt eine direkte Berührung zwischen der Elektrode des Instruments und dem Gewebe vor.

**[0016]** - Falls die Widerstandskenngröße größer als der Widerstandsschwellwert, die Funkengröße jedoch kleiner als der Funkengrößenschwellwert ist, besteht kein Funke oder der Funke schlägt in die Luft.

**[0017]** - Die Widerstandskenngröße ist größer als der Widerstandsschwellwert und zugleich ist die Funkenkenngröße größer als der Funkengrößenschwellwert, so liegt eine Einwirkung des Funkens oder Plasmas auf das Gewebe ohne Metallberührung vor.

Zur Berechnung oder sonstigen Ermittlung der Widerstandskenngröße und der Funkenkenngröße können alle geeigneten Verfahren angewendet werden. Beispielsweise kann die Widerstandskenngröße als der lineare Anteil des elektrischen Widerstands festgelegt sein, der sich als Quotient eines Kennwerts für den gemessenen Strom und eines Kennwertes der gemessenen Spannung ergibt. Insbesondere kann der Quotient aus den Effektivwerten von Strom und Spannung gebildet und dann mit dem Leistungsfaktor multipliziert werden, um die Widerstandskenngröße zu bilden. In dieser Widerstandskenngröße ist nicht nur der Gewebewiderstand enthalten. Vielmehr kommen weitere Anteile, beispielsweise der Leitungswiderstand der Zuleitung des Instruments und gegebenenfalls auch lineare Widerstandsanteile aus dem Funken hinzu. Jedoch ist die so ermittelte Widerstandskenngröße ein gutes Maß für den Gewebewiderstand.

Zur Ermittlung der Funkenkenngröße kann der nichtlineare Anteil des Stroms bestimmt werden, der maßgeblich auf den von dem Funken gebildeten nichtlinearen Widerstand zurückgeht. Dazu kann die Analyseeinrichtung darauf eingerichtet sein, anhand eines linearen Ersatzschaltbilds und der gemessenen Spannung (bzw. des entsprechenden Kennwerts der Spannung) den zugehörigen Strom $i_{sim}(t)$ zu berechnen. Die Differenz $i_f(t)$ zwischen dem berechneten Strom $i_{sim}(t)$ und dem gemessenen Strom $i(t)$ kennzeichnet den von der Nichtlinearität des Funkens hervorgerufenen Strom. Das Verhältnis aus dem Effektivwert $F_{eff}$ dieser Differenz $i_f(t)$ und dem Effektivwert $i_{eff}$ des gemessenen Stroms $i(t)$ kann als Funkenkenngröße Frei genutzt werden.

Anstelle des Vergleichs der Widerstandskenngröße mit dem Widerstandsschwellwert und des Vergleichs der Funkenkenngröße mit dem Funkengrößenschwellwert ist es auch möglich, die Widerstandskenngröße und die Funkenkenngröße auf schnelle zeitliche Veränderungen hin zu untersuchen. Übersteigt die Steilheit des Anstiegs der Funkenkenngröße dFrei/dt sowie des Anstiegs der Widerstandskenngröße dR/dt jeweils einen bestimmten Grenzwert $f_0$, $r_0$, kann wiederum auf Metallberührung geschlossen werden.

Einzelheiten von Ausführungsformen der Erfindung ergeben sich aus der Zeichnung, Ansprüchen sowie insbesondere der Beschreibung. Es zeigen:

Figur 1 ein von einem Generator gespeistes funkenbildendes, elektrochirurgisches Instrument bei der Einwirkung auf biologisches Gewebe, in schematisierter Veranschaulichung.

Figur 2 Komponenten des Generators des Instruments und des biologischen Gewebes als Schaltbild.

Figur 3 ein Blockschaltbild der Einrichtung zur Metallerkennung.

Figur 4 und 5 von der Einrichtung nach Figur 3 genutzte Ersatzschaltungen.

Figur 6 ein Ablaufschema für die Metallerkennung auf Basis von Widerstandsschwellwert und Funkengrößenschwellwert und

Figur 7 ein Ablaufschema zur Metallerkennung auf Basis der zeitlichen Veränderung der Widerstandskenngröße und der Funkenkenngröße.

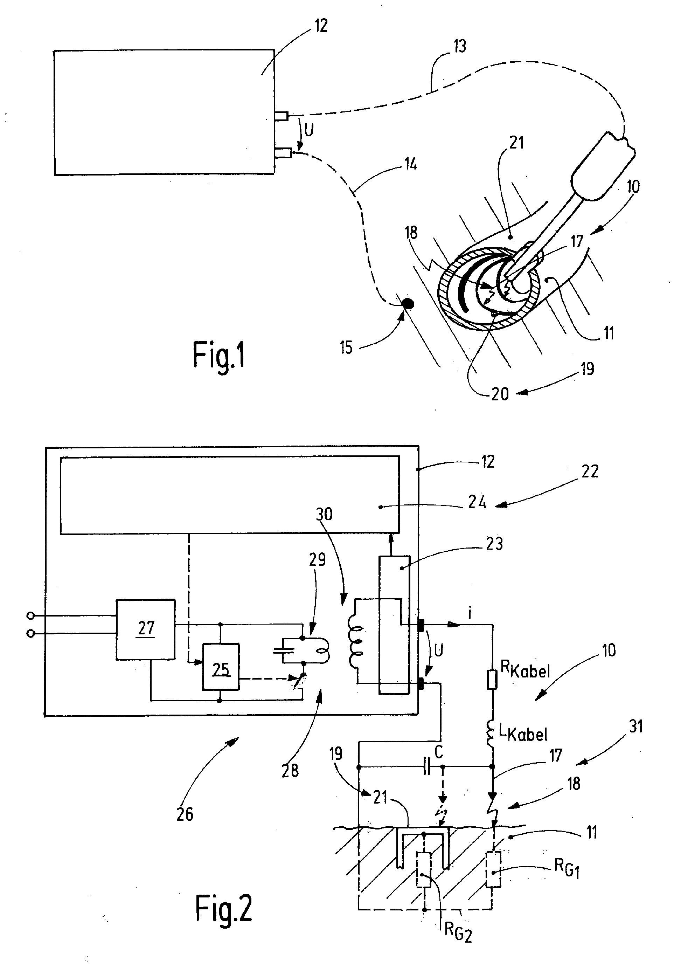

In Figur 1 ist schematisch eine Anwendungssituation veranschaulicht, bei der mittels eines elektrochirurgischen Instruments 10 an biologischem Gewebe 11 eine Behandlung durchgeführt wird. Das Instrument 10 wird dazu von einem Gerät 12 mit elektrischem Strom versorgt. Die von dem Gerät 12 bereitgestellte Spannung u sowie der zu dem Instrument 10 gelieferte Strom i sind vorzugsweise periodische Größen mit einer Frequenz von vorzugsweise mehreren 100 kHz, zum Beispiel 350 kHz. Obwohl die vorliegende Erfindung darauf nicht beschränkt ist, eignet sie sich insbesondere für monopolare Anwendungen. Entsprechend führt eine erste Leitung 13 von dem Gerät 12 zu dem Instrument 10. Eine zweite Leitung 14 führt von dem Gerät 12 zu einer großflächigen, an der unversehrten Oberfläche des Gewebes 11, insbesondere der Haut des Patienten, angebrachten Neutralelektrode 15.

Das Instrument 10 weist mindestens eine Elektrode 17 auf, von der ein Stromfluss zu dem biologischen Gewebe 11 ausgeht. Je nach aktueller Situation kann der Strom durch direkten Gewebekontakt oder über einen Funken 18 fließen, der zwischen der Elektrode 17 und dem biologischen Gewebe 11 überschlägt. Der Funken 18 kann dabei je nach Anwendungsfall ein Volumen durchqueren, das Luft und/oder Wasserdampf und/oder Dampf anderer Flüssigkeiten wie Purisole, Kochsalzlösung und/oder ein sonstiges Gas wie Stickstoff, Kohlendioxid oder Edelgas wie insbesondere Argon enthält. Das vorhandene Gas oder Gasgemisch oder Dampf ionisiert im Bereich des Funkens 18 und bildet ein Plasma, wobei der Funke das biologische Gewebe 11 berührt und Strom in dieses einleitet.

Das biologische Gewebe 11 kann elektrisch leitende Fremdkörper, insbesondere Metallteile 19 enthalten, wie insbesondere Stents 20 (Figur 1) zum Offenhalten von Hohlgefäßen 21 wie dem Ösophagus oder dergleichen. Das Metallteil 19 kann außerdem eine Klammer, eine Schraube, Platte, ein Draht oder ein sonstiges in den Körper eines Patienten eingebrachtes Bauteil sein.

Bei der Vornahme von Behandlungen des biologischen Gewebes 11 kann es vorkommen, dass der Funke 18 Metallteile 19 berührt. Eine solche Berührung soll keinesfalls unkontrolliert erfolgen. Sie kann erwünscht sein, beispielsweise um Metallteile gezielt zu erwärmen oder zu durchtrennen, beispielsweise um Stents zu kürzen oder Operationspinzetten zu erwärmen, beispielsweise um eine Gewebekoagulation zwischen den Branchen der Pinzette hervorzurufen. Sie kann jedoch auch unerwünscht sein, beispielsweise wenn eine Erwärmung von Metallteilen 19 zur Schädigung derselben sowie zur Schädigung umliegenden biologischen Gewebes führen würde. Bei schlechter Sicht auf das Anwendungsgebiet, ist es für den Anwender jedoch manchmal schwierig, Metallteile rechtzeitig zu erkennen. Insbesondere kann es jedoch schwierig sein, die Berührung der Metallteile 19 durch den Funken 18 schnell genug zu erkennen.

Zur Erkennung von Metallberührung durch den Funken 18 ist ein Metalldetektor 22 vorgesehen, der, wie in Figur 2 dargestellt, Teil des Geräts 12 oder alternativ auch Teil des Instruments 10 oder als Zwischenmodul ausgebildet sein kann. Ein solches Zwischenmodul ist anstelle der Leitungen 13, 14 an das Gerät 12 anzuschließen, wobei die Leitungen 13, 14 dann an das Zwischenmodul angeschlossen werden.

Zu dem Metalldetektor 22 kann eine Messeinrichtung 23 gehören, die die für das Instrument 10 bereitgestellte Spannung u sowie dem zu dem Instrument 10 gelieferten Strom i erfasst. Sie erfasst dabei mindestens einen Kennwert Ku der Spannung sowie mindestens einen Kennwert Ki des Stroms. Ein solcher Kennwert Ki des Stroms kann der Augenblickswert i(t) der Spitzenwert $i_{peak}$, der Mittelwert $i_{mean}$, der Effektivwert $i_{eff}$ oder ein anderer zur Charakterisierung geeigneter Wert sein. Solche Kennwerte können kontinuierlich oder in abgetasteter Weise erfasst werden. Beim Abtasten von Augenblickswerten wird vorzugsweise mit mehr als doppelter Frequenz des Stroms abgetastet (zum Beispiel mit mehr als 700 kHz). Als Kennwert Ku für die Spannung kann gleichfalls der Augenblickswert u(t), der Spitzenwert $u_{peak}$, der Mittelwert $u_{mean}$, der Effektivwert $u_{eff}$ oder ein anderer zur Charakterisierung geeigneter Wert erfasst werden. Die Erfassung kann kontinuierlich oder periodisch erfolgen. Bei Abtastung des Augenblickswerts wird vorzugsweise mit mehr als der doppelten Frequenz der Spannung abgetastet. Wenn im Nachfolgenden und im Vorstehenden von der "Erfassung des Stroms" oder "Erfassung der Spannung" gesprochen wird, bezieht sich dies auf die erläuterte Erfassung eines entsprechenden Kennwerts des Stroms oder der Spannung.

Die gemessenen Kennwerte Ku, Ki für die Spannung und den Strom werden von der Messeinrichtung 23 an eine Analyseeinrichtung 24 übergeben. Die Analyseeinrichtung dient dazu, anhand der Kenngrößen für den Strom und die Spannung zu unterscheiden, ob der Funken 18 mit dem Gewebe 11 oder mit dem Metallteil 19 in Berührung steht.

Die Analyseeinrichtung 24 kann im Weiteren mit einer Steuerung 25 eines Generators 26 verbunden sein, der in Figur 2 schematisch veranschaulicht ist und zur Speisung des Instruments 10 mit hochfrequenter elektrischer Energie dient. Der Generator 26 umfasst typischerweise neben der Steuereinrichtung 25 ein Netzteil 27 und einen daran ange—

schlossenen Leistungsoszillator 28 mit einem Schwingkreis 29 und einer potentialfreien HF-Auskoppelspule 30. Die Steuerung 25 kontrolliert den Betrieb des Generators 26, d.h. sie aktiviert und deaktiviert diesen, gibt die Spannung und/oder den Strom und/oder die Leistung und/oder den Crestfaktor desselben vor. Die Steuerung 25 kann mit nicht weiter veranschaulichten Bedienorganen kommunizieren, die als Schalter oder Einstellvorrichtungen an dem Gerät 12 und/oder dem Instrument 10 und/oder als weitere gesonderte Schalter oder Eingabeeinrichtungen ausgebildet sind.

Um anhand des erfassten Stroms und der erfassten Spannung zu erkennen, ob der Funke 18 das Gewebe 11 oder das Metallteil 19 berührt, ist die Analyseeinrichtung 24 dazu eingerichtet, mindestens zwei Kenngrößen zu bestimmen, nämlich eine Funkenkenngröße $F_{rel}$ und eine Widerstandskenngröße R. Als Funkenkenngröße $F_{rel}$ wird vorzugsweise eine Kenngröße ausgewählt, die die Größe und/oder die Intensität des Funkens 18 kennzeichnet. Als Funkenkenngröße $F_{rel}$ kommt beispielsweise eine Kenngröße in Frage, die die Nichtlinearität des von dem Instrument 10, dem Funken 18 und dem biologischen Gewebe 11 gebildeten elektrischen Netzwerks kennzeichnet. Das so gebildete elektrische Netzwerk ist in vereinfachter Form in Figur 2 veranschaulicht. Zu ihm gehören der ohmsche Widerstand $R_{Kabel}$ der Leitungen 13, 14, die Induktivität $L_{Kabel}$, die zwischen den Leitungen 13, 14 zu messende Kapazität C sowie der Gewebewiderstand $R_{G1}$ oder $R_{G2}$. Der Gewebewiderstand $R_{G1}$ ist der Widerstand des biologischen Gewebes, wenn der Funken 18 auf dieses trifft. Der Gewebewiderstand $R_{G2}$ ist der typischerweise geringere Gewebewiderstand, der sich durch die Stromverteilung des Metallteils 19 ergibt, wenn der Funken 18 auf dieses trifft. Weiter gehört zu dem Netzwerk der nichtlineare Widerstand des Funkens 18.

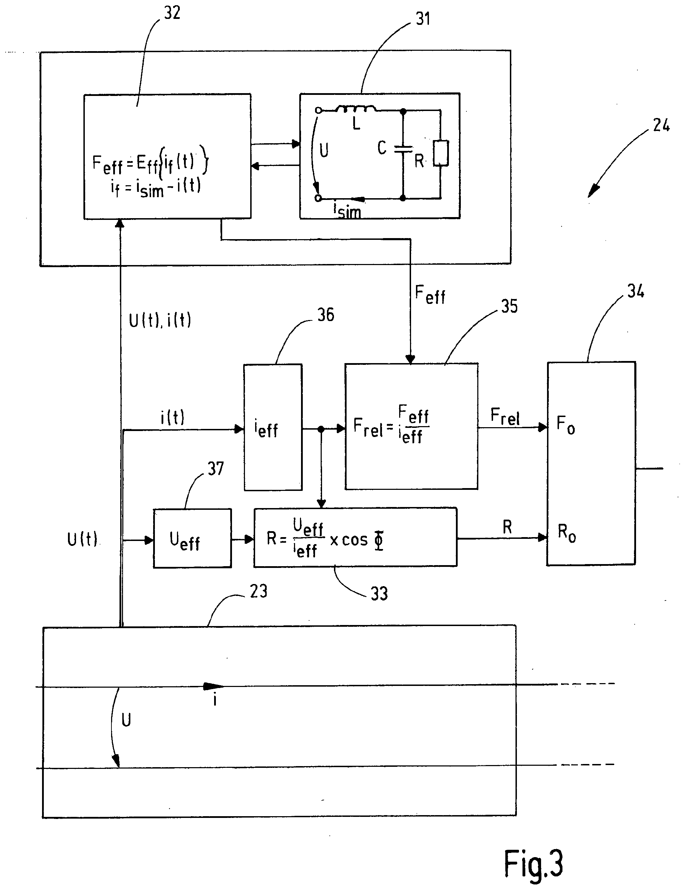

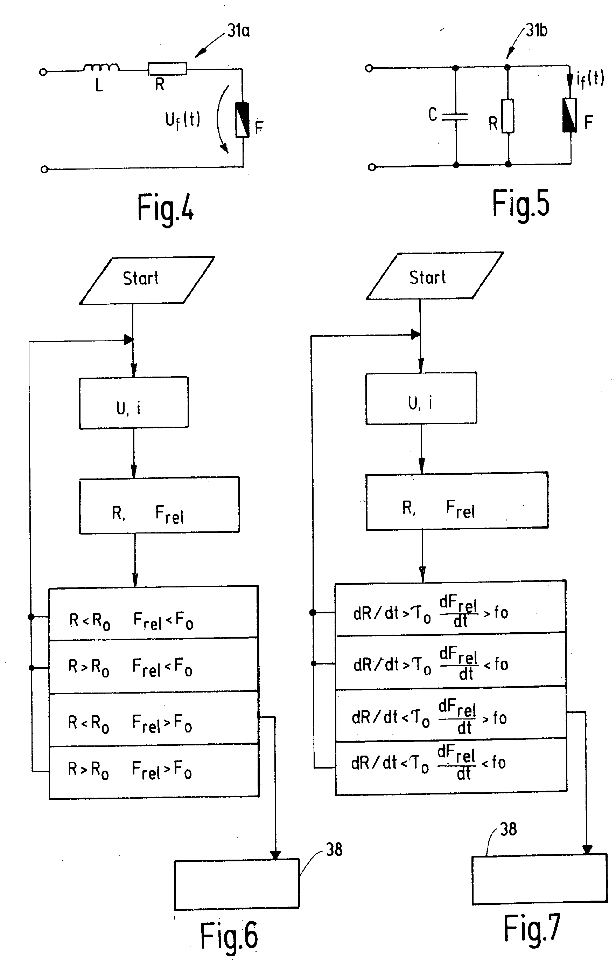

Die Analyseeinrichtung 24 kann über eine interne Ersatzschaltung 31, ein internes Netzwerkmodell 31 dieses elektrischen Netzwerks verfügen, wie in Figur 3 angedeutet ist. Das Netzwerkmodell 31 kann eine vereinfachte Repräsentation des tatsächlich nach Figur 2 vorhandenen Netzwerks sein, bei dem die auftretenden Induktivitäten, Kapazitäten und Widerstände zu Elementen R, L, C zusammengefasst sind. Je nachdem, ob sich das Netzwerk aktuell vorwiegend induktiv oder vorwiegend kapazitiv verhält, kann anstelle des Netzmodells 31 ein vereinfachtes Netzmodell 31a oder ein 31b gemäß Figur 4 oder Figur 5 gewählt werden. Die Auswahl kann die Analyseeinrichtung anhand der Nach- oder Voreilung des Stroms i zur Spannung u treffen und entscheiden.

Die Analyseeinrichtung 24 ist dazu ausgelegt, zunächst die Werte der Elemente L, R, C des Netzwerkmodells 31, 31a, 31b zu bestimmen. Dazu werden die Werte der Elemente L, R, C zum Beispiel im Rahmen einer Regressionsrechnung oder mit der Methode des kleinsten Fehlerquadrats so festgelegt, dass die im Netzwerkmodell 31, 31a, 31b rechnerisch auftretenden Ströme und Spannungen bestmöglich an den tatsächlich erfassten Strom und die tatsächlich erfasste Spannung angepasst sind. Nachdem der ohmsche Leitungswiderstand $R_{Kabel}$ meist weniger als 1 Ohm beträgt, ist dieser vernachlässigbar. Damit entspricht der Wert R des Widerstands nach dem Netzwerkmodell 31a,b nach Figur 4 oder 5 dem Gewebewiderstand. Die mit dem linearen Netzmodell nicht in Einklang bringenden Anteile des Stroms i und der Spannung u werden dem nichtlinearen Widerstand F des Funkens 18 zugeordnet. Die Bestimmung der Werte der Elemente L, R, C kann zu Beginn einer Aktivierung, oder in Zeitabständen oder kontinuierlich erfolgen.

Aus dem realen Strom i(t) und dem durch das lineare Netzwerk vorgegebenen Strom $i_{sim}$(t) lässt sich wie in Figur 3 angedeutet die Funkenkenngröße $F_{rel}$ bestimmen. Figur 3 veranschaulicht dazu mehrere Blöcke zur Veranschaulichung der Verarbeitung des gemessenen Stroms i(t) und der Spannung u(t). Diese Blöcke können durch Programmcode oder anderweitig realisiert sein. Die Zuordnung von Funktionen zu Blöcken ist rein beispielhaft und kann auch anderweitig getroffen sein.

Ein Block 32 bestimmt sporadisch, periodisch oder kontinuierlich die Elemente R, L und/oder C des Netzwerkmodells 31 nach Figur 3, 4 oder 5. Das Netzwerkmodell 31 berechnet dann mit dem Input der gemessenen Spannung u(t) einen Strom $i_{sim}$(t) sowie dessen Differenz $i_f$(t) zum gemessenen Strom i(t) und dessen Effektivwert Feff von $i_f$(t). Der Strom $i_{sim}$(t) stimmt, insbesondere wenn der Funke 18 gezündet hat, nicht mit dem Effektivwert $i_{eff}$ des gemessenen Stroms i(t) überein.

Der Stromfehler $i_f$(t) berechnet sich als Differenz des aus dem Ersatzschaltbild nach Figur 4 oder 5 für die HF-chirurgische Anwendung berechneten Sollstroms $i_{sim}$(t) und des während der HF-Applikation gemessenen HF-Stroms i (t) :

$$i_f(t) = i_{sim}(t) - i(t)$$

Block 32 errechnet die Abweichung des simulierten Stroms $i_{sim}$(t) vom gemessenen Strom i(t) als Stromfehler $i_f$(t). Alternativ kann auch $i_f$(t) anhand der Augenblickswerte des Sollstroms $i_{sim}$(t) und des tatsächlichen Stroms i(t) errechnet werden. $F_{eff}$ ist der Effektivwert des Stromfehlers $i_f$(t):

$$F_{eff} = \sqrt{\frac{1}{T} \cdot \int_T i_f^2(t)}$$

und wird in Block 32 berechnet.

Der Stromfehler $i_f(t)$ wird maximal, wenn der gemessene HF-Strom $i(t)$ von dem berechneten Regressionsstrom maximal abweicht. Diese Abweichung ist vor allem bei HFchirurgischen Anwendungen mit Funkenbildung der Fall, bei dem es aufgrund der Funkenbildung zu einer starken Verzerrung des HF-Stroms $i(t)$ kommt. Im Fall der Funkenbildung und der damit einhergehenden Verzerrung des Stroms ist der nichtlineare Anteil des Ersatzschaltbilds zur Berechnung des Regressionsstroms besonders hoch. Die linearen Elemente des Ersatzschaltbilds nach Figur 3 bis 5 können den gemessenen HF-Strom $i(t)$ nicht vollständig erklären und führen zu einem von dem gemessenen HF-Strom $i(t)$ abweichenden Regressionsstrom $i_{sim}(t)$. Dies korreliert mit einem hohen Stromfehler $i_f(t)$, wodurch der effektive Funkenkennwert $F_{eff}$ entsprechend ansteigt. Durch Verhältnisbildung mit dem gemessenen effektiven HF-Strom $i_{eff}$ wird in Block 35 ein relatives Maß für die Funkenbildung erhalten.

Die Analyseeinrichtung 24 berechnet, wie oben erläutert, anhand der Regressionsanalyse die linearen Werte des Netzwerkmodels 31 nach Figur 3 bzw. 31a .oder b nach Figur 4 oder 5. Der ohmsche Anteil R kann als Widerstandskenngröße genutzt werden, die hauptsächlich den Widerstand des biologischen Gewebes 11 kennzeichnet, d.h. in der Darstellung nach Figur 2 dem Gewebewiderständen $R_{G1}$ oder $R_{G2}$ zuzuordnen ist. Die Regressionsrechnung dazu wird von dem Block 32, dem Regressionsblock fortwährend durchgeführt, um während des gesamten Betriebs des Instruments 10 aktuell die Widerstandskenngröße R zu bestimmen.

Alternativ kann der Gewebewiderstand auch, wie in Figur 3 veranschaulicht, durch einen gesonderten Widerstandsrechenblock 33 bestimmt werden. Dazu bestimmen die Blöcke 36, 37 zunächst aus den Augenblickswerten $i(t)$ und $u(t)$ die Effektivwerte der HF-Spannung und des HF-Stroms $u_{eff}$ und $i_{eff}$, sowie den Leistungsfaktor $cos\Phi$. (Alternativ kann der Widerstandsrechenblock 33 diese Effektivwerte auch von der Messeinrichtung 23 erhalten.) Als Widerstandskenngröße bildet der Widerstandsrechenblock 33 den Quotienten des Effektivwerts $u_{eff}$ der HF-Spannung $u(t)$ und des Effektivwerts $i_{eff}$ multipliziert mit dem Leistungsfaktor $cos\Phi$.

Ein Komparator 34 vergleicht die in Block 35 ermittelte Funkenkenngröße $F_{rel}$ mit einem Funkengrößenschwellwert $F_0$. Außerdem vergleicht der Komparator die Widerstandskenngröße R mit einem Widerstandsschwellwert Ro. Den Ablauf veranschaulicht Figur 6. Die Funkenkenngröße $F_{rel}$ kann größer oder kleiner sein als der Funkengrößenschwellwert $F_0$. Ebenso kann die Widerstandskenngröße R größer oder kleiner sein als der Widerstandsgrößenschwellwert Ro. Daraus ergeben sich vier mögliche Konstellationen. Es hat sich gezeigt, dass bei geeigneter Wahl von Ro zu beispielsweise 300 Ohm und $F_0$ zu beispielsweise 0,4 die Berührung von Metall durch den Funken 18 dann gegeben ist, wenn die Widerstandskenngröße R kleiner als der Widerstandsschwellwert $R_0$ und die Funkenkenngröße Frei größer als der Funkengrößenschwellwert $F_0$ ist. Bei den anderen drei Konstellationen liegen andere Situationen vor, zum Beispiel Kontaktanwendung zwischen Instrument 10 und Gewebe 11 ohne Funkenbildung, Aktivierung der Elektrode 17 in Luft ohne gezündetem Funken oder Funke zum Gewebe 11 mit Funkenbildung. Wird hingegen ein gegen ein Metallteil gezündeter Funke erfasst, wird zu einem Block 38 verzweigt, der eine entsprechende Maßnahme symbolisiert. Eine solche Maßnahme kann die Aussendung eines wahrnehmbaren Signals oder eine Beeinflussung der Steuerung 25 sein. Diese kann beispielsweise die Leistung reduzieren oder erhöhen oder den Generator 26 abschalten, den Crestfaktor ändern oder dergleichen.

Figur 7 veranschaulicht eine abgewandelte Ausführungsform des Verfahrens. Es werden wiederum laufend, wie vorstehend beschrieben, die Widerstandskenngröße R und die Funkenkenngröße $F_{rel}$ bestimmt. Anders als im vorstehend beschriebenen Verfahren werden diese jedoch nicht mit absolutem Schwellwerten $F_0$ und $R_0$ verglichen. Vielmehr wird als Auswertekriterium die zeitliche Veränderung $dR/dt$ der Widerstandskenngröße R und die zeitliche Veränderung $dF_{rel}/dt$ der Funkenkenngröße $F_{rel}$ ermittelt und mit Grenzwerten ro und $f_0$ verglichen. Zu dem Maßnahmenblock 38 wird wiederum dann verzweigt, wenn die Veränderung $dR/dt$ des Gewebewiderstands unter einem Widerstandsänderungsschwellwert ro und die Veränderung $dF_{rel}/dt$ der Funkenkenngröße $F_{rel}$ größer als ein Funkengrößenänderungsschwellwert $f_0$ ist. Damit gelingt es, falsche Rückschlüsse auf Metallberührung durch stochastische Messwertschwankung zu vermeiden.

Eine erfindungsgemäße Einrichtung zur Metallerkennung bei funkenbildenden elektrochirurgischen Instrumenten 10 enthält einen Metalldetektor 22, der anhand des zu dem Instrument 10 gelieferten Stroms $i(t)$ (sowie der Spannung) unterscheidet, ob ein von dem Instrument 10 ausgehender Funke 18 auf biologisches Gewebe 11 oder ein Metallteil 19 trifft. Vorzugsweise erfolgt dies durch Bestimmung desjenigen Anteils des Stroms $i(t)$, der nicht zu einer linearen Ersatzschaltung 31 passt. Die Elemente der linearen Ersatzschaltung 31 werden zuvor oder während des Betriebs in einer Regressionsrechnung bestimmt. Aus dem Strom $i(t)$ wird als erstes Entscheidungskriterium die Funkenkenngröße $F_{rel}$ bestimmt. Als zweites Entscheidungskriterium wird eine Widerstandskenngröße R bestimmt, die den Gewebewiderstand kennzeichnet. Beide Kenngrößen werden mit Schwellwerten Fo, Ro verglichen. Unterschreitet der Gewebewiderstand R den Widerstandsschwellwert $R_0$ und überschreitet die Funkenkenngröße $F_{rel}$ den Funkengrößenschwellwert Fo, wird ein Signal erzeugt, das die Aktivierung des Funkens 18 gegen ein Metallteil 19 kennzeichnet.

Bezugszeichen:

| | |
|---|---|
| 10 | Instrument |
| 11 | biologisches Gewebe |
| 12 | Gerät |
| 13, 14 | Leitungen |
| 15 | Neutralelektrode |
| u | für das Instrument 10 bereitgestellte Spannung |
| i | zu dem Instrument 10 gelieferter Strom |
| 16 | |
| 17 | Elektrode |
| 18 | Funken |
| 19 | Metallteil |
| 20 | Stent |
| 21 | Hohlgefäß |
| 22 | Metalldetektor |
| 23 | Messeinrichtung |
| | |
| Ki | Kennwert für den Strom i |
| i (t) | Augenblickswert des gemessenen Stroms |
| $i_{mean}$ | Mittelwert des Stroms |
| $i_{eff}$ | Effektivwert des Stroms |
| $i_{peak}$ | Spitzenwert des Stroms |
| $i_f(t)$ | Differenz zwischen dem berechneten Strom und dem gemessenen Strom |
| Ku | Kennwert für die Spannung |
| u(t) | Augenblickswert der gemessenen Spannung |
| $u_{mean}$ | Mittelwert der Spannung |
| Ueff | Effektivwert der Spannung |
| $U_{peak}$ | Spitzenwert der Spannung |
| $u_f(t)$ | Differenz zwischen der berechneten Spannung und der gemessenen Spannung |
| 24 | Analyseeinrichtung |
| 25 | Steuerung |
| 26 | Generator |
| 27 | Netzteil |
| 28 | Leistungsoszillator |
| 29 | Schwingkreis |
| 30 | HF-Auskoppelspule |
| Frei | Funkenkenngröße (Verhältnis zwischen $F_{eff}$ und $i_{eff}$ (t)) |
| R | Widerstandskenngröße |
| $R_{Kabel}$ | Ohmscher Leitungswiderstand |
| $L_{kabel}$ | Kabelinduktivität |

(fortgesetzt)

| C | Kapazität |
|---|---|
| $R_G$, $R_{G1}$, $R_{G2}$ | Gewebewiderstand |
| 31, 31a, 31b | Ersatzschaltung, Netzwerkmodell |
| R, L, C | Elemente des Ersatzschaltbildes |
| F | Funkenwiderstand |
| $i_{sim}(t)$ | mit dem Netzwerkmodell errechneter Strom |
| T | Periodendauer |
| $F_{eff}$ | Effektivwert des Stromfehlers |
| $i_{eff}$ | Effektivstrom |
| 32 | Regressionsblock |
| 33 | Widerstandsrechenblock |
| $F_0$ | Funkengrößenschwellwert |
| Ro | Widerstandsschwellwert |
| 34 | Komperatorblock |
| 35 - 38 | Blöcke |
| $f_0$ | Funkengrößenänderungsschwellwert |
| $r_0$ | Widerstandsänderungsschwellwert |
| cos Φ | Leistungsfaktor |

**Patentansprüche**

1. Einrichtung zur Metallerkennung bei der Einwirkung auf biologisches Gewebe (11) mittels eines funkenbildenden elektrochirurgischen Instruments (10),

   mit einer Messeinrichtung (23) zur Erfassung einer von einem Generator (26) zum Betrieb des Instruments (10) bereitgestellten Spannung (u) und zur Erfassung des von dem Generator (26) zu dem Instrument (10) gelieferten Stroms (i),
   mit einem Metalldetektor (22), der dazu eingerichtet ist, anhand des Stroms (i) und der Spannung (u) zu unterscheiden, ob ein von dem Instrument (10) ausgehender Funke (18) auf biologisches Gewebe (11) oder ein Metallteil (19) trifft, **dadurch gekennzeichnet, dass** der Metalldetektor (22) aufweist:

   eine Analyseeinrichtung (24), die zur Ermittlung einer vom Gewebewiderstand ($R_G$) abhängigen Widerstandskenngröße (R) und einer von der Funkengröße abhängigen Funkenkenngröße ($F_{rel}$) eingerichtet ist, und
   einen Komparator (34), der zum Vergleich der Widerstandskenngröße (R) mit einem Widerstandsschwellwert ($R_0$) und der Funkenkenngröße ($F_{rel}$) mit einem Funkengrößenschwellwert ($F_0$) eingerichtet ist, wobei der Komparator (34) darauf

   eingerichtet ist, eine Berührung von Metall durch einen von dem Instrument (10) ausgehenden Funken (18) anzuzeigen, wenn:

   - die Widerstandskenngröße (R) den Widerstandsschwellwert ($R_0$) unterschreitet und
   - die Funkenkenngröße ($F_{rel}$) den Funkengrößenschwellwert ($F_0$) überschreitet.

2. Einrichtung nach einem der vorstehenden Ansprüche, **dadurch gekennzeichnet, dass** der Generator (26) ein HF-Generator ist.

3. Einrichtung nach einem der vorstehenden Ansprüche, **dadurch gekennzeichnet, dass** die Messeinrichtung (23) darauf eingerichtet ist, den mindestens einen Kennwert (Ki, Ku) von Strom (i) und Spannung (u) fortlaufend zu bestimmen.

4. Einrichtung nach Anspruch 3, **dadurch gekennzeichnet, dass** der Kennwert (Ki, Ku) von Strom (i) und Spannung (u) der Augenblickswert ($i(t)$, $u(t)$), der Spitzenwert ($i_{peak}$, $u_{peak}$), Mittelwert ($i_{mean}$, $u_{mean}$), der Effektivwert ($i_{eff}$, $u_{eff}$) oder ein anderer zur Charakterisierung geeigneter Wert ist.

5. Einrichtung nach einem der vorstehenden Ansprüche, **dadurch gekennzeichnet, dass** die Messeinrichtung (23) darauf eingerichtet ist, den von Strom (i) und Spannung (u) festgelegten Leistungsfaktor ($\cos \Phi$) zu bestimmen.

6. Einrichtung nach Anspruch 1, **dadurch gekennzeichnet, dass** die Analyseeinrichtung (24) darauf eingerichtet ist, die Funkenkenngröße (Frei) anhand des nichtlinearen Anteil des Stroms (i) zu bestimmen.

7. Einrichtung nach Anspruch 6, **dadurch gekennzeichnet, dass** die Analyseeinrichtung (24) darauf eingerichtet ist, einen Schätzwert für den nichtlinearen Anteil des Stroms (i) zu bestimmen.

8. Einrichtung nach Anspruch 7, **dadurch gekennzeichnet, dass** die Analyseeinrichtung (24) darauf eingerichtet ist, den Schätzwert für den nichtlinearen Anteil des Stroms (i) anhand der Abweichung des gemessenen Strom (i) von einem anhand einer linearen Ersatzschaltung (31) errechneten Stroms ($i_{sim}$) zu bestimmen.

9. Einrichtung nach Anspruch 8, **dadurch gekennzeichnet, dass** die Analyseeinrichtung (24) darauf eingerichtet ist, die Werte der Elemente der linearen Ersatzschaltung (31) aus dem gemessenen Strom (i) und der gemessenen Spannung (u) zu bestimmen.

**Claims**

1. Device for metal detection when acting on biological tissue (11) by means of a spark-forming electrosurgical instrument (10),

   with a measuring device (23) for detecting a voltage (u) provided by a generator (26) for operation of the instrument (10), and for detecting the current (i) supplied to the instrument (10) by the generator (26),
   with a metal detector (22) which is configured to distinguish, on the basis of the current (i) and voltage (u), whether a spark (18) emitted by the instrument (10) meets biological tissue (11) or a metal part (19),
   **characterised in that** the metal detector (22) comprises:

   an analysis device (24) which is configured to determine a resistance parameter (R) dependent on the tissue resistance ($R_G$) and a spark parameter ($F_{rel}$) dependent on the spark size, and
   a comparator (34) which is configured to compare the resistance parameter (R) with a resistance threshold value ($R_0$) and compare the spark parameter ($F_{rel}$) with a spark size threshold value ($F_0$),

   wherein the comparator (34) is configured to indicate a contact of metal by a spark (18) emitted by the instrument (10) when:

   - the resistance parameter (R) is less than the threshold resistance value ($R_0$) and
   - the spark parameter ($F_{rel}$) exceeds the spark size threshold value ($F_0$).

2. Device according to one of the preceding claims, **characterised in that** the generator (26) is an HF generator.

3. Device according to one of the preceding claims, **characterised in that** the measurement device (23) is configured to determine the at least one characteristic value (Ki, Ku) of the current (i) and voltage (u) continuously.

4. Device according to claim 3, **characterised in that** the characteristic value (Ki, Ku) of the current (i) and voltage (u) is the momentary value ($i(t)$, $u(t)$), the peak value ($i_{peak}$, $u_{peak}$), the mean value ($i_{mean}$, $u_{mean}$), the effective value ($i_{eff}$, $u_{eff}$) or another value suitable for characterisation.

5. Device according to any of the preceding claims, **characterised in that** the measurement device (23) is configured to determine the power factor (cos φ) established by the current (i) and voltage (u).

6. Device according to claim 1, **characterised in that** the analysis device (24) is configured to determine the spark parameter ($F_{rel}$) using the non-linear part of the current (i).

7. Device according to claim 6, **characterised in that** the analysis device (24) is configured to determine an estimated value for the non-linear part of the current (i).

8. Device according to claim 7, **characterised in that** the analysis device (24) is configured to determine the estimated value for the non-linear part of the current (i) from the deviation of the measured current (i) from a current ($i_{sim}$) calculated using a linear equivalent circuit (31).

9. Device according to claim 8, **characterised in that** the analysis device (24) is configured to determine the values of the elements of the linear equivalent circuit (31) from the measured current (i) and the measured voltage (u).

**Revendications**

1. Dispositif de détection de métal lors d'une action sur des tissus biologiques (11) au moyen d'un instrument électrochirurgical (10) formant des étincelles,

   comprenant un dispositif de mesure (23) destiné à mesurer une tension (u) fournie par un générateur (26) en vue du fonctionnement de l'instrument (10), et à mesurer le courant (i) délivré par le générateur (26) à l'instrument (10),
   comprenant un détecteur de métal (22) qui est conçu pour distinguer à l'aide du courant (i) et de la tension (u), si une étincelle (18) émanant de l'instrument (10) rencontre des tissus biologiques (11) ou un élément en métal (19), **caractérisé en ce que** le détecteur de métal (22) présente :

   un dispositif d'analyse (24) qui est conçu pour déterminer une grandeur caractéristique de résistance (R), dépendant de la résistance des tissus ($R_G$), et une grandeur caractéristique d'étincelle ($F_{rel}$) dépdendant de la taille des étincelles, et
   un comparateur (34) qui est conçu pour comparer la grandeur caractéristique de résistance (R) avec une valeur seuil de résistance ($R_0$) et la grandeur caractéristique d'étincelle ($F_{rel}$) avec une valeur seuil de grandeur d'étincelle ($F_0$),

   le comparateur (34) étant conçu pour indiquer un contact avec du métal par une étincelle (18) émanant de l'instrument (10), si :

   - la grandeur caractéristique de résistance (R) passe en dessous de la valeur seuil de résistance ($R_0$), et
   - la grandeur caractéristique d'étincelle ($F_{rel}$) dépasse la valeur seuil de grandeur d'étincelle ($F_0$).

2. Dispositif selon l'une des revendications précédentes, **caractérisé en ce que** le générateur (26) est un générateur HF.

3. Dispositif selon l'une des revendications précédentes, **caractérisé en ce que** le dispositif de mesure (23) est conçu pour déterminer en continu la caractéristique (Ki, Ku), au nombre d'au moins une, du courant (i) et de la tension (u).

4. Dispositif selon la revendication 3, **caractérisé en ce que** la caractéristique (Ki, Ku) du courant (i) et de la tension (u) est la valeur instantanée (i(t), u(t)), la valeur de crête ($i_{peak}$, $u_{peak}$), la valeur moyenne ($i_{mean}$, $u_{mean}$), la valeur effective ($i_{eff}$, $u_{eff}$) ou une autre valeur appropriée pour la caractérisation.

5. Dispositif selon l'une des revendications précédentes, **caractérisé en ce que** le dispositif de mesure (23) est conçu pour déterminer le facteur de puissance (cos Φ) défini par le courant (i) et la tension (u).

6. Dispositif selon la revendication 1, **caractérisé en ce que** le dispositif d'analyse (24) est conçu pour déterminer la caractéristique de grandeur d'étincelle ($F_{rel}$) à l'aide de la fraction non linéaire du courant (i).

7. Dispositif selon la revendication 6, **caractérisé en ce que** le dispositif d'analyse (24) est conçu pour déterminer une valeur estimée pour la fraction non linéaire du courant (i).

8.  Dispositif selon la revendication 7, **caractérisé en ce que** le dispositif d'analyse (24) est conçu pour déterminer la valeur estimée pour la fraction non linéaire du courant (i) à l'aide de l'écart du courant (i) mesuré par rapport à un courant ($i_{sim}$) calculé à l'aide d'un circuit équivalent linéaire (31)

9.  Dispositif selon la revendication 8, **caractérisé en ce que** le dispositif d'analyse (24) est conçu pour déterminer les valeurs des éléments du circuit équivalent linéaire (31) à partir du courant (i) mesuré et de la tension (u) mesurée.

Fig.1

Fig.2

12

Fig.3

Fig.4

Fig.5

Fig.6

Fig.7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20120215213 A1